# EUROPEAN PATENT SPECIFICATION

(11) **EP 1 241 982 B1**
(45) Date of publication and mention of the grant of the patent: **18.10.2006**
(21) Application number: 00988143.4
(22) Date of filing: 19.12.2000
(51) Int. Cl.: A61B 5/00

(54) **INTEGRATED SOFTWARE SYSTEM FOR IMPLANTABLE MEDICAL DEVICE INSTALLATION AND MANAGEMENT**
INTEGRIERTES SOFTWARESYSTEM ZUR INSTALLATION UND VERWALTUNG IN IMPLANTIERBAREN MEDIZINISCHEN VORRICHTUNGEN
SYSTEME LOGICIEL INTEGRE DESTINE A L'INSTALLATION ET A LA GESTION D'UN DISPOSITIF MEDICAL IMPLANTABLE

(30) Priority: 24.12.1999 US 173082 P
(43) Date of publication of application: 25.09.2002
(73) Proprietor: MEDTRONIC, INC., Minneapolis, Minnesota 55432-5604 (US)
(72) Inventor: MERRY, Randy, L., Maple Grove, MN 55311 (US); HODGES, Andrew, C., Blaine, MN 55434 (US); LINBERG, Kurt, R., Eden Prairie, MN 55346 (US)
(74) Representative: Hughes, Andrea Michelle
(86) International application number: PCT/US2000/034425
(87) International publication number: WO 2001/047410

(56) References cited:
- WO-A-97/18639
- WO-A-98/42407
- WO-A-99/60926
- US-A- 5 619 991
- US-A- 5 752 976
- US-A- 5 833 623
- US-A- 5 911 687

## Description

### FIELD OF THE INVENTION

The present invention relates to implantable medical devices (IMDs). Specifically, the invention relates to systems implemented to enhance productivity and accuracy during the implant of the IMDs and further to integrate patient data for adaptation to third-party databases or a central data management system. The central data management system may be a web-enabled remote server which stores device registration and patient management data.

### BACKGROUND OF THE INVENTION

After the implantation of an IMD, for example, a cardiac pacemaker, clinician involvement with respect to the IMD has typically only begun. The IMD usually cannot be merely implanted and forgotten, but must be monitored for optimal results, and may require adjustment of certain parameters or settings, or even replacement, in response to or in anticipation of changes in patient condition or other environmental factors, or based on factors internal to the device. IMDs may also contain logic devices such as digital controllers, which may need to undergo firmware or software upgrades or modifications. In addition, information about the IMD may be gathered for treatment or research purposes. For example, many IMDs are capable of storing certain state information or other data regarding their operation internally.

In addition to the patient concerns described above, the implantation of a medical device is an event which must be carefully documented or recorded by various clinicians and commercial entities. For example, a clinician may wish to record information about the device such as its serial and model number in order to inform the patient of any firmware or software updates or upgrades involving the device, and to issue reminders to the patient regarding significant dates involving the IMD in order to generally aid in patient compliance. The IMD may also have a regular maintenance period suggested or prescribed, for example, for renewal of a power supply or refill of a reservoir containing a drug administered by the device. Similarly, the manufacturer and/or seller of the device will probably wish to record information about the device such as its serial and model number, manufacturing date, its batch or lot, the patient receiving the implant, the clinical entity administering the device, and the like, in order to ensure than any important information that may involve the device may be promptly provided to the patient either directly or indirectly. In addition, the manufacturer may be engaged in demographic or cohort clinical studies or data collection regarding etiological and device outcome scenarios across a population receiving a certain medical device or general category of medical device. Furthermore, the manufacturer may wish to track demand of various product lines in order to determine which products or types of products are subject to greater demand, and accordingly should receive a greater investment of health care research and supply funds. In particular, the manufacturer will wish to maximize the likelihood that an implantable medical device will be available to a patient that needs one.

In addition, the manufacturer of a medical device will wish to promptly invoice medical devices that have been implanted, so that the devices may be paid for if they have not been paid for by the time of implant. Accordingly, upon implantation, it would be desirable to provide information of the implant event to various business departments of the manufacturer and clinic, including, for example, the accounts receivable and accounts payable offices, the finance department, research and development, marketing, or assembly/fabrication departments.

Because of the importance of information regarding device implementation like that discussed above, it is desirable that such information be carefully memorized and tracked to help ensure positive patient outcome and compliance. Accordingly, an advancement that would increase the accuracy or efficiency of this recording process would be welcomed.

Further, it is a typical medical practice to keep an accurate record of past and contemporaneous procedures relating to an IMD uplink with, for example, an IMD programmer, i.e. a device capable of making changes to the firmware or software of an IMD. It is typically desired that the report contain the identification of all the medical devices involved in any interactive procedure. Specifically, all peripheral and major devices that are used in downlinking to the IMD may be reported. Currently, such procedures are manually reported, and require an operator or a medical person to manually enter data during each procedure. One of the limitations of such manual reporting procedures is the possibility for human error in data entry, thus motivating rechecking of the data to verify accuracy. Generally, the use of human clinicians and technicians to analyze data and implement changes in device therapy can result in inefficiencies and errors.

Yet a further condition of the prior art relates to the interface between a human operator and a programmer system. To aid a patient in the administration of a deployed medical device, clinicians such as pacing clinicians may be made available to implement desirable changes in the treatment regimen effected by an IMD. Generally, a medical device manager/technician should be trained on the clinical and operational aspects of the programmer. Under current practices, a technician may attend a class or training session sponsored by a clinic, a hospital, or the manufacturer to successfully manage a programmer-IMD procedure. Further, ideally the operator will keep abreast of new developments and new procedures in the management, maintenance and upgrade of the IMD. Accordingly, it is desirable that operators of programmers, IMDs, and related medical devices receive regular training or information about the IMDs they work with. This information will preferably be widely distributed, because IMDs, programmer devices, and related medical devices are distributed throughout the world. Further, the number of people having implanted medical devices has been increasing over the last few years, with an attendant increase in operator personnel. The total effect of these phenomenon is a widely dispersed and large body of operators. Thus, it is desirable to have a high efficiency communications system that would enhance data communications, both between the IMDs and medical instruments, such as programmers; and between operators and entities providing IMD updates and education such as manufacturers. However, despite any improvement in clinician communication and training that may be effected, it may be desirable to automate device administration, maintenance, and upgrading to the extent possible in order to ensure that device instructions and data are appropriate to the situation. Furthermore, it would be desirable to limit the degree to which human and particularly clinician involvement is required to effect the communication between an IMD and a remote resource. Furthermore, it may be desirable to limit clinician, technician, or other human involvement in certain appropriate aspects of IMD deployment within a patient, once the IMD is implanted. For example, after implantation, the device implanted must be registered. This registration may be linked to the device's host patient, or may be anonymous. In addition, various documents relating to insurance, regulatory, business and administrative forms tracking the implantation must be completed. Other forms may be used to effect inventory management and control supply flow.

Further, it may be preferred to have an operable communication between the various implants to provide a coordinated clinical therapy to the patient. Thus, there is a need to monitor the IMDs and the programmer on a regular, if not a continuous, basis to ensure optimal patient care. In the absence of other alternatives, this imposes a great burden on the patient if a hospital or clinic is the only center where the necessary upgrade, follow up, evaluation and adjustment of the IMDs could be made. Further, even if feasible, the situation would require the establishment of multiple service areas or clinic centers to support the burgeoning number of multi-implant patients worldwide. Document US-A-5 752 976 discloses a system according to the preamble of claim 1.

### SUMMARY OF THE INVENTION

Based on the motivations and problems summarized above, it is desirable to have a programmer unit that would connect to a centralized data source and repository. This repository may be termed, for example, a Device Registration/Patient Management System, or a remote data center. This remote data center will preferably provide access to an expert system allowing for downloading of upgrade data or other information to a local, i.e., IMD environment. Further, it may be desirable to have an integrated software system for IMD installation, registration, cataloging and invoicing, and also for efficient voice and data communications to transfer information between the IMDs and a remote expert data center for dispensation of therapy and clinical care on a real-time basis.

In order to improve productivity and accuracy with respect to device registration and documentation, and in general the memorization of an implant event, e. g., implant of an implantable pacemaker, defibrillator, or other medical device, an embodiment of present invention integrates the IMD's software or firmware application with that of a programmer device in order to effect automatic record keeping and administrative tasks and supply resulting data to a centralized repository and distribution lender, which may be termed a Device Registration and Patient Management Center, or DRPMC. In addition, certain software used in conjunction with computers outside the IMD is used to aid in the correct implantation of an implantable pacemaker or defibrillator. Under the prior art, typically each activity that takes place during an implant is an independent, and often manual, procedure. This invention, in contrast, would integrate these activities within a common programmer. During an implant procedure of an implantable medical device, or IMD, such as pacemaker or defibrillator, an analytical program is used to determine the placement and function of the device. Using the analytical program, the clinician executes sensing and threshold tests. Under an embodiment of the present invention, the results of these tests, and other data is automatically transferred from the analytical program to the implantable device application and a server application running on a DRPMC. A programming application is used to setup initial system parameters of the device at implant and to setup the initial patient data that is stored in each implantable device. In addition, a computerized DRPMC application is used to integrate the information from the analytical program and device application, with additional device registration and patient management data. Data from the Device Registration/Patient Management Application running on the DRPMC may be optionally transmitted into other affiliated or third-party databases systems for use in accounts payable, device registration, patient management, inventory management, accounts receivable systems, supplier systems and other business systems.

The removal of human errors in device registration, implant forms and inventory management improves the overall quality of the system, productivity is enhanced by removing physical steps from the system, i.e. human data entry or manual recordation, and replacing them with integrated and/or automatic systems.

Further, in one embodiment of the present invention, it is possible to enable the gathering of high resolution diagnostic/physiologic data, and to transfer information between the IMDs and a remote data center to dispense therapy and clinical care on real-time basis. Further, the data system contemplated by the present invention enables an efficient system for data storage, collection and processing to effect changes in control algorithms of the IMDs and associated medical units to promote real-time therapy and clinical care.

The proliferation of patients with multi-implant medical devices worldwide has made it imperative to provide remote services to the IMDs and timely clinical care to the patient. The use of programmers and related devices to communicate with the IMDs and provide various remote services has become an important aspect of patient care. In addition to the instant invention, the use of programmers may be implemented in a known manner.

In light of the disclosures of prior art references, the present invention provides a vital system delivering efficient therapy and clinical care to the patient as well as streamlining administrative and business processes for clinicians and their supply chain.

In a representative embodiment of the instant invention, one or more IMDs, such as a pacemaker, defibrillator, drug pump, neurological stimulator, physiological signal recorder may be deployed in a patient This IMD may be equipped with a radio frequency transmitter or receiver, or an alternative wireless communication telemetry technique or media which may travel through human tissue. For example, the IMD may contain a transmission device capable of transmitting through human tissue such as radio frequency telemetry, acoustic telemetry, or a transmission technique that uses patient tissue as a transmission medium. Alternatively, an IMD may be deployed in a fashion by which a transmission or receiving device is visible externally to the patient but is connected directly or via wires to the IMD. An external device, which may generally be termed a programmer, may be positioned outside the patient, the programmer being equipped with a radio frequency or other communication means compatible with the communication media of the IMD or the IMD transmitter/receiver, which may be external to the IMD and may further be external to the patient. In an illustrative embodiment of the subject invention, the programmer contains a radio frequency transmitter/receiver or similar radio frequency telemetry device. Communication may be effected between the IMD transmitter/receiver and the external programmer, e.g. via radio frequency. The programmer will be connected via a wireless or physical communication media, e.g. via modem and direct dial connection, with a data network, LAN, WAN, wireless, or infrared network. In an alternative embodiment of the subject invention, the programmer may have a direct connection or tunneled connection directly to the DRPMC. In yet another alternative embodiment of the subject invention, the system may be implemented as a data network that allows the programmer access to the DRPMC from many locations, for example providing for a programmer that is portable.

The amount of historical data, particularly patient-specific historical data used as input to control systems can be virtually unlimited when it is stored externally to the patient. Furthermore, a more thorough comparison can be made between patients with similar diseases as data and therapy direction are centralized, which may be expected to result in gains to the body of medical knowledge and treatment efficacy. Data from other medical systems, either implanted or external, such as etiological databases, can be incorporated easily into the DRPMC. Other anonymous patient experiences or treatment data may be more quickly incorporated into a subject patient's IMD regime than might be possible with existing systems of IMD programming or upgrading. In addition, a subject patient's own historical treatment parameters and corresponding outcomes may be used in making IMD programming and other treatment decisions.

The instant invention provides IMD clinicians and manufacturers with access to virtually unlimited computing power as part of their data collection and therapy calculation processes.

According to an embodiment of the present invention, IMD administration, management and manipulation will be more efficient and efficacious. The present invention may be effected, in part, by the provision of a programmer device, which may be a standalone device or a computer peripheral device, that is capable of connecting an IMD, or simply data telemetrically received from an IMD, to a network or other data communication link. While the interface between the programmer and a DRPMC is referred to generally herein as a "Network Interface", or the like, it will be appreciated to those skilled in the art that the interface may serve as an interface to a variety of data communications systems, including not only networks, but also, without limitation, direct dial-up connections, dedicated lines, direct satellite links, and other non-network data communications connections.

In a preferred embodiment of the subject invention, a host patient, i.e., a patient having an IMD implanted within, presents themselves to a programmer. This programmer will preferably have the capability of communicating with the IMD via wireless means, such as by radio transmissions. In one embodiment of the subject invention, the programmer may be available in a clinical setting for use by several patients in a treatment facility such as a hospital, nursing home, or ambulatory care center.

In one embodiment of the invention, data can be interrogated from a device, possibly with the aid of a Device Registration/Patient Management System computer , by a programmer in a clinical setting and then uploaded to an information network to which the Device Registration/Patient Management System is connected. This information network may be according to any network protocol, for example, TCP/IP over the Internet. The uploading to a central interrogation computer may also be effected over a direct dial-up connection or a dedicated line. Upon uploading of the data, a medical professional or other clinician may be alerted to the fact the data has been uploaded. This clinician may then view the data, if desired.

In addition, the Device Registration/Patient Management System may transmit information regarding an implant, such as the device registration implant information, to the systems of the accounting, inventory, finance, and any other interested business unit of the device manufacturer, supplier, or other interested entities which may wish to track the information about implantation of the IMD. As an example, businesses in the supply chain for medical devices may adjust inventory levels, production orders or quotas. If the account of the implanting clinician or entity warrants for example, additional inventory will be shipped to the account if the clinician or entity has indicated a desire to maintain a set inventory level of devices on hand. In addition, an invoice for payment may be automatically generated by a business system computer and mailed via U.S. mail to clinician or implanting entity, or third party payer for payment. In an alternative scenario, payment or invoice information may be sent to various third-party computer systems according to the protocols of various agreed data third party payer electronically via electronic data interchange (EDI) agreements or arrangements. For example, information about a number of implantation events or patients may be supplied as a batch to a business partner or third party periodically, rather than on a one-by-one basis as the implant information is received. Information about the implantation of the medical device may also be supplied by means of a data interface to third-party medical record systems.

In an alternative embodiment, the programmer may be implemented in part as a software client which may run on a computer remotely from the DRPMC server. Preferably, either the programmer, the Device Registration/Patient Management System program or device is capable of autonomously and dynamically determining the model of an IMD, for example, according to manufacturer, type, and model number, as well as the specific serial number of a particular device. When an IMD is within communication range of a programmer, the Device Registration/Patient Management System of the present invention is also preferably capable of configuring a deployed IMD, or commanding the programmer to retrieve data from the IMD.

The invention is defined in claim 1. Any embodiment which is in contradiction to the subject-matter of claim 1 is not part of the invention.

### BRIEF DESCRIPTION OF THE DRAWINGS

Figure 1 depicts a general architecture diagram of a system embodying the subject invention.
Figure 2 depicts the general architecture an implementation of the system of Figure 1 using a typical network.
Figure 3 depicts the architecture of a broader system of the present invention, including third party systems.

### DETAILED DESCRIPTION OF THE DRAWINGS

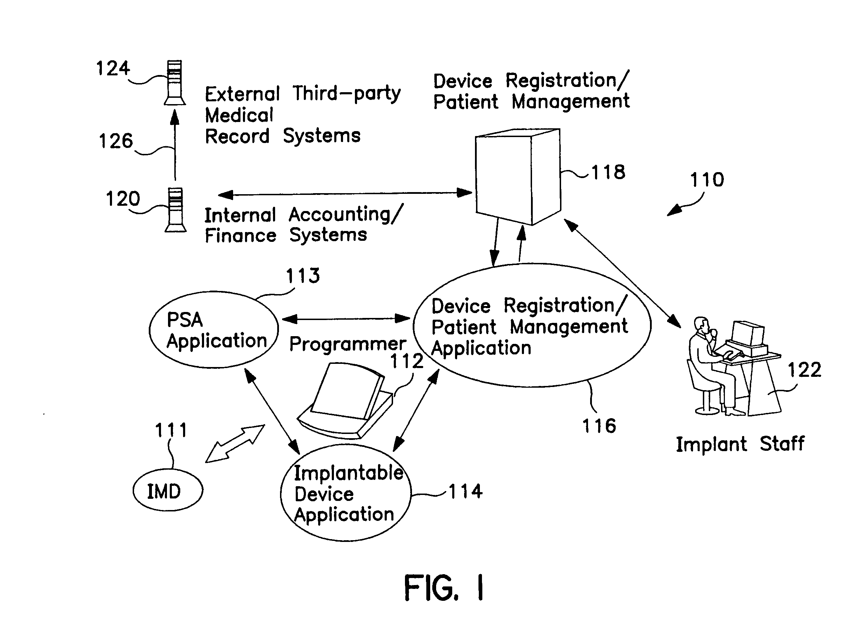

Figure 1 depicts a general architecture diagram of a system embodying the subject invention. The system 110 provides for a unified administration and recording system for implant information applicable to a number of unique patients. A representative embodiment of the subject invention will be discussed with an example implant that is a pacer replacement implant. However, the present invention is suitable for a number of existing IMDs, for example, a pacemaker, defibrillator, drug pump, neurological stimulator, physiological signal recorder, oxygen sensor, or the like. An abstract IMD is depicted as element 111. For example, a type of software suitable for integration according to an embodiment of the present invention is the pacing system analyzer (PSA) software used in implantation of an implantable pacemaker or defibrillator running on a programmer 112. The application is depicted as architecture element 113 in Figure 1.

In contrast to implantation procedures of the prior art, in which each administrative activity that takes place during and after an implant are independent procedures, this invention would integrate these activities within a common programmer and its modules. In the example of a pacemaker or defibrillator, during an implant procedure a PSA 113 is used to determine the proper lead placement. Using the PSA 113, the clinician executes sensing and threshold tests, then the data is automatically transferred from the PSA application 113 to the Device Registration/Patient Management Application 116 running on DRPMC 118. An Implantable Device Application 114 is also running on programmer 112. For example, a pacemaker application, a type of Implantable Device Application, is used to setup initial system parameters of a pacemaker device at implant and to setup the initial patient data that is stored in each implantable device 111. In addition, a computerized Device Registration/Patient Management application 116, running preferably on DRPMC 118, is used to integrate the information from the PSA 113 and pacemaker application 114, with additional device registration and patient management data obtained from programmer 112. Data from the Device Registration/Patient Management Center 118 is transmitted into DRPMC-affiliated processing system 120 for use in accounts payable, device registration, patient management, inventory management and accounts receivable systems.

In the illustrative example of a pacer replacement implant, the implanting clinician staff 122 queries the DRPMC 118 prior to implant and requests information on the patient and device/lead information. Data, potentially including, but not limited to, device serial number, model number, lead manufactures, lead compatibility data, implant notes, and patient name and contact information are communicated to the programmer 112. In a scenario in which the device is a new device rather than a replacement device, query of the DRPMC 118 is preferably not required or prompted prior to the implant procedure. However, during pacer replacement, pertinent patient and IMD data may be provided to properly-authenticated users 122 by DRPMC 118.

During the implant process, for example, in the scenario of a pacer replacement implant, after the leads have been placed the clinician may use the pacing system analyzer 113, or comparable implant testing application resident on the programmer 112 to confirm proper implant placement and/or functioning. For example, in a pacer replacement embodiment, a pacing system analyzer application may use sensing and threshold tests, in order to confirm correct lead placement.

Following the tests for proper implant placement and function, the results or data from the device tests may be forwarded to at least the DRPMC 118 on which the Device Registration/Patient Management application 116 runs, and the device application 114 that is run on the programmer 112 or IMD 111 itself. For example, in the pacer replacement, the sensing and threshold values that were determined during the test sequence are forwarded to the DRPMC 118 and pacemaker application 114.

In a preferred embodiment, an interaction between a deployed IMD 111 and a programmer 112 or Device Registration/Patient Management System 118 may take place within a discrete session. This session may encompass interrogation of one or more IMDs deployed in a single patient. A session according to the present invention may proceed according to the following scenario. In order to begin an interrogation session, a host patient will typically present to a programmer. For example, the patient may place themselves in the vicinity of the programmer 112 within range of the telemetry capacities of the programmer. For example, this may take place at a medical facility or clinical setting such as an Emergency Room, Follow-up Clinic or Operating Room. At the initiation of a session, it will be preferable to configure the target IMD 111 for optimal operation for remote interrogation. For example, the programmer 112 may be programmed to issue a command to the target IMD 111 to "Cancel Magnet", "Resume Therapy," or another command to enter a mode consistent with the interrogation process.

Either prior to or after the establishment of a telemetry or other communication link with the target IMD 111, the Programmer Operator will effect a communications link between the programmer 112 and the remote DRPMC computer 118. This Programmer Operator may be a human attendant or technician, but preferably will be an automated module of the programmer firmware or software, or may be implemented as a software application on a general purpose computer connected to the programmer. Alternatively, the Device Registration/Patient Management System computer 118 may lead a human or automated programmer operator through the steps of establishing a telemetry interface between the IMD 111 and programmer 112; with the programmer 112 in turn notifying the Device Registration/Patient Management System 118 when a telemetry connection has been established.

Communication with the remote interrogation server may be established via a network connection, such as a LAN or WAN or over a public network. In this embodiment of the present invention in which the programmer is preferably attended by an operator, the operator may be the host patient of the target IMD, or it may be attendant personnel at a clinical setting. In either case, the operator may connect the programmer to a suitable network connection, if a network connection is not already in place. For example, a direct dial-up connection may be established in this manner by physically connecting the programmer 112 into a telephone connection jack such as a RJ-11 analog jack. The operator at some point would turn the programmer 112 on and cause the programmer 112 system to dial a pre-configured telephone number. Alternatively, other, more continuous types of network connections may also be established.

Following the transmission of the test results to the DRPMC 118, the device application on the programmer 112 completes the programming of the implant. For example, the programmer 112 may make appropriate changes to the parameter values, diagnostics settings and patient data stored within the implanted device. Thereafter, the implanting clinician or other personnel may transmit the implant data in the DRPMC 118 to a corresponding client application on the programmer 112. This information may contain additional information on the device, leads, patient, clinician, institution, and other related data.

At the completion of the implant procedure, the programmer 112 communicates with the central DRPMC 118 via standard communication methods such as by a dial-up connection or other direct connection, or via a network such as the Internet. The central DRPMC 118 system acts as a central clearinghouse or router of relevant data presented by the programmer 112. For example, the DRPMC 118 may transmit information regarding an implant, such as the device registration implant information, to the systems 120 of the accounting, inventory, finance, and any other interested business unit of the device manufacturer, supplier, or other interested entities which may wish to track the information about implantation of the IMD. Accordingly, DRPMC 118 may supply certain information or data to external third-party systems 124. This data may be supplied over a public network, as will be discussed below, or may be supplied via direct connection or electronic data interchange (EDI) system 126. As an example, businesses in the supply chain for medical devices may adjust inventory levels, production orders or quotas. If the account of the implanting clinician or entity warrants, for example, additional inventory will be shipped to the account if the clinician or entity has indicated a desire to maintain a set inventory level of devices on hand. In addition, an invoice for payment may be automatically generated by a business system computer 120 and mailed via U.S. mail to clinician or implanting entity 122, or third-party payer for payment. In an alternative scenario, payment or invoice information may be sent to various third-party computer systems 124 according to the protocols of various agreed data third party payer electronically via electronic data interchange (EDI) agreements or arrangements. For example, information about a number of implantation events or patients may be supplied as a batch to a business partner or third party periodically, rather than on a one-by-one basis as the implant information is received. Information about the implantation of the medical device may also be supplied by means of a data interface to third-party medical record systems.

Other third parties to which aggregate or singular device demand and consumption information may be supplied include parts suppliers to the IMD manufacturer or supplier. In a preferred embodiment, the DRPMC 118 system provides for automated ordering of necessary raw materials, parts, or other supplies periodically according to manufacturer need compiled within the DRPMC 118. In a preferred embodiment of the subject invention, such data communications to third parties are effected through DRPMC 118, in order to centralize the security of a manufacturer computer network.

In addition to providing information, including payment information, to third party payers or other third-party systems 124, the invoice is preferably communicated to the local clinician's office system 122 to begin the payment procedure. For example, the payment information may be sent as a SMTP e-mail or UDP to the clinician or clinic over the Internet or other publicly accessible network. For example, a direct connection, dial-up connection, or dedicated line may be used to convey payment information from internal accounting system 120 to clinician system 122. In a preferred embodiment of the subject invention, the financial system or other business system of the implant manufacturer may be transmitted to the DRPMC system 118, in order for forwarding to clinician system 122. It will be appreciated to those skilled in the art that the centralized nature of the DRPMC 118 provides a centralized security point and interface with external systems at which security measures such as firewalls or proxy servers may be implemented.

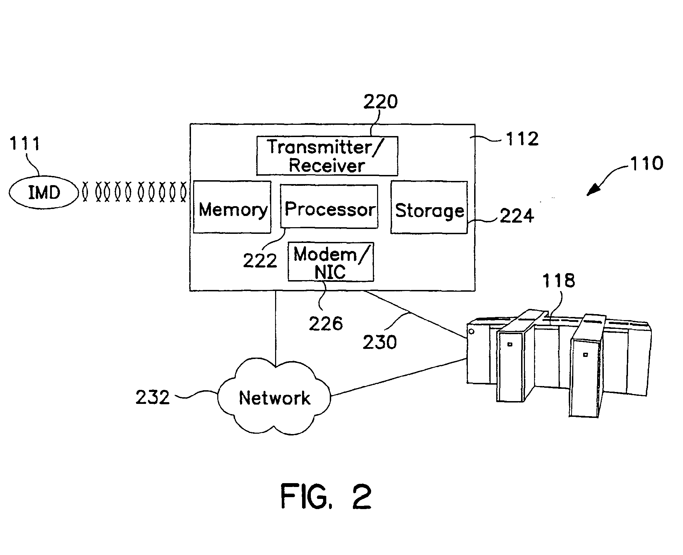

Figure 2 depicts in greater detail a suitable architecture for programmer 112 of Figure 1. As shown in Figure 2, programmer 112 contains a transmitter/receiver 220, a processor 222, storage device 224, and communication device 226. Communication device 226 may be, for example, a modem or network interface card. It may be seen in Figure 2 that programmer 112 contains architecture components similar to those seen in a computer, and in an alternate embodiment of the subject invention, the communication system 110 of the present invention may be deployed with programmer 112 implemented as a computer with a peripheral device that may communicate with IMD 111.

While in an illustrative embodiment, several programmers 112 share a single DRPMC 118, alternative embodiments may have a single or multiple programmers 112 communicating with distributed or parallel computers, in addition to or in place of DRPMC 118.

While programmer 112 is portrayed primarily as a self-contained or standalone unit, it will be appreciated that programmer 112 may also be implemented as a peripheral transmitter receiver capable of wireless communication with IMD 111, and also in communication with a computer such as a personal computer, laptop or portable computer. Implemented on a computer, programmer 112 may also be a terminal or client of a remote computer, including of DRPMC 118. It will be appreciated that in the event that programmer 112 is implemented as a peripheral end terminal, some of the components of programmer 112, e.g., storage component 224 may be implemented on DRPMC 118 or a storage device accessible to DRPMC 118 rather than in the terminal implementing programmer 112.

As shown in Figure 2, communications between programmer 112 and DRPMC 118 may be effected either through a network 232, such as a LAN or the Internet, or communications may be effected through a direct dial-up or dedicated line, or through a terminal connection to a mainframe. These possible implementations are indicated generally by direct communications link 230. Typically, these connections may be considered alternatives; or both communications links, i.e., relatively direct link 230 and link through network 232 may be implemented in order to provide a backup communications system to the link used as the primary communication method.

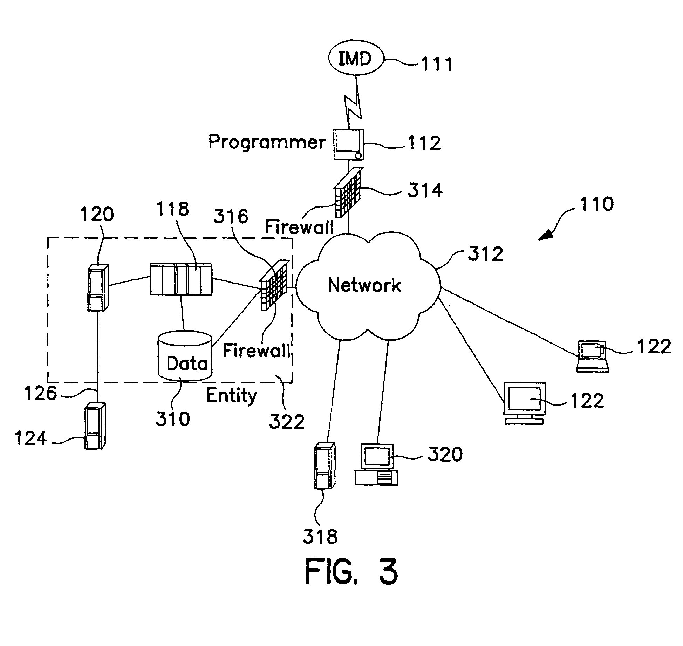

Figure 3 depicts the architecture of a broader system of the present invention, including third party systems. As shown, information network 110 includes Device Registration/Patient Management System computer 118. Device Registration/Patient Management System 118 will preferably be possessed of appreciably more computing power than possible with IMD 111, in terms of processor speed, RAM available, and other data storage. For example, some commercially-available personal computers may contain sufficient computing power to operate as a server capable of carrying out many data storage and processing tasks of the present invention. In a preferred embodiment of the subject invention, however, DRPMC 118 will be a mainframe, multi-processor supercomputer, or a multi-processor workstation, such as a type available from Silicon Graphics, Inc./SGI of Mountain View, California.

Regardless of which computing device is used, in accordance with the present invention, the computing device will be configured as a server capable of communicating directly or indirectly with programmer 112. The DRPMC 118 will preferably have sufficient storage, either internal to the computer or linked to the computer, for the storage of massive amounts of historical data from, for example, a particular patient having an IMD 111 in communication with DRPMC 118, and/or subject data from relevant physiologic studies or from demographic or market groups having similar medical conditions and/or deployed IMDs. For example, as depicted in Figure 3, DRPMC 118 is linked to or contains data storage element 310. Data storage element 310 may contain any suitable means of data storage, including but not limited to hard drive, or another readable/writable magnetic or optical storage. In a preferred embodiment of the subject invention, data storage element 310 has a redundant array of disks such as a redundant array of inexpensive disks (RAID) system.

Security and integrity of the patient information and IMD interface operation will preferably be closely guarded for at least the following reasons: First, patient physiologic data detected by a deployed IMD 111 will be transmitted via programmer 112 to DRPMC 118 for purposes of analysis of this data, and treatment regimens and/or IMD 111 instructions, firmware, or software may be changed on the basis of this information. Accordingly, integrity of transmitted data and instructions will preferably be maintained so as to avoid adverse patient outcomes or patient outcomes that do not take full advantage of the subject invention. In addition, patient information that may be linked to an identifiable individual is typically regarded as confidential. Accordingly, encryption or tunneling will preferably be provided to ensure patient confidentiality, particularly when transmissions between programmer 112 and DRPMC 118 takes place though media other than a dedicated line/direct dial-up connection, such as connection 230 in Figure 2. For example, transmissions may be effected over a packet-based network technology over a public network or internetwork 232. For example, if the transmissions are routed over the Internet using TCP/IP, encryption will preferably be used. As an alternative to encryption, a proprietary data exchange format/interface that is kept secret may be used in communications between IMD 111 and computer DRPMC 118. However, even with secure dedicated lines 230 or a secret data format, digital signatures will preferably be used to detect corruption of data. Additional implementations of security systems may also be utilized in accordance with the subject invention, including biometric security apparatus and methods to detect inalterable physical characteristics of persons attempting to access the patient data in order to authenticate the would-be user of the system.

Security measures such as the foregoing will preferably be used to authenticate the programmer 112 and IMD 111, as well as persons attempting to access patient data such as clinician system 122. Accordingly, a preferred embodiment of the subject invention utilizes digital signatures and encryption of the patient information and IMD 111 instructions being transmitted according to the present invention. Encryption of patient information will serve to protect patient confidentiality. Each transmission of patient data will preferably have a digital signature that can be checked against the transmission payload to ensure that patient data and IMD 111 instructions were not corrupted during transmission. Examples of encryption/digital signature schemes that should prove sufficient for suitable encryption of patient information and digital signatures include PGP, the RSA public key infrastructure scheme, or other consumer-level or higher, prime number based encryption signature scheme. Biometric data used to authenticate and verify accessors of the data may include retina scans, iris scans, fingerprint scans, veinprint scans, voiceprints, characteristics, facial geometry/facial recognition according to facial nodal points, or hand geometry scans.

In addition to the above security implementations, a preferred embodiment of the subject invention incorporates firewall and/or proxy server technology, as indicated in Figure 3 at firewalls 314 and 316. Such security measures not only protect patient data stored in data storage element 310 from access by unauthorized persons, but also protect programmer 112 and IMD 111 from improper snooping and/or improper instruction from negligent or unscrupulous persons that may have access to data network 312.

The data network is depicted generally at network cloud 312. For example, communication may be effected by way of a TCP/IP connection, particularly one using the Internet, as well as a PSTN, DSL, ISON, Cable Modem, LAN, WAN, MAN, direct dial-up connection, a dedicated line, or a dedicated terminal connection to a mainframe. Various persons, entities, or systems including clinicians 122, third-party payers system 318, or supplier system 320 may access the patient's data from information network 312 or direct link 230 if access is so permitted by entity 322. Entity 322 may be, for example, an IMD manufacturer or distributor.

Transmissions between an IMD 111 and programmer 112 will also preferably be protected from transmission errors using similar encryption, authentication, and verification techniques to those discussed above, and/or wireless communication enhancement techniques such as wireless modulation or another suitable wide-frequency spectra technique. Preferably, encryption and/or authentication will be effected end-to-end, i.e., covering the entire transmission from IMD 111 to DRPMC 118, rather than effecting one encryption/verification scheme between IMD 111 and programmer 112, and a different scheme between programmer 112 and DRPMC 118. As an alternative to, or in addition to the above authentication scheme, radio frequency pulse coding, spread spectrum, direct sequence, time-hopping, frequency hopping, a hybrid spread spectrum technique, or other wireless modulation techniques may be employed in order to reduce interference between IMD 111 and other IMD or other wireless devices, and to generally offer improved accuracy, reliability, and security to transmissions between IMD 111 and programmer 112, may be used to avoid cross-talk or confusion among IMDs and/or programmers in proximity to each other. For example, radio coding may be implemented to avoid transmission errors or device confusion between neighboring IMD 111 patients utilizing a device implementing aspects of the present invention in a managed-care setting.

Upon establishment of a network connection, or direct dial-up connection, a communications link is established over which the programmer 112 may establish a connection with the DRPMC 118. The initial communication may focus on authentication of the programmer 112. This will preferably include verification that the programmer 112 is certified for interrogating IMDs, i.e., a verification process has established that the software and hardware revisions are current, and that the authentication information uniquely identifies a specific known programmer 112.

Further considering the steps in a representative embodiment of the invention in which the programmer 112 is attended by an operator, the DRPMC 118 may next instruct the programmer 112 Operator such as a human user how to configure the telemetry interface between programmer 112 and IMD 111. This would typically be specific for the type of IMD 111 being interrogated and might involve, for example, placing a programming head or wand near the IMD 111, or just positioning the patient and programmer 112 in proximity. The programmer 112 may then notify the DRPMC 118 that a telemetry connection has been established.

Upon establishment of a telemetry interface, preferably DRPMC 118 selects a proper software module to interrogate the programmer 112 in light of specific IMD 111 being administered.

Upon execution of the applicable software module, the DRPMC 118 may retrieve certain pertinent data from the programmer 112 and/or IMD 111, including physiologic data regarding the host patient stored power remaining, amount of drug remaining within the device, or hardware, software, or firmware version information, or other device status information.

Upon completion of the data retrieval operation, the DRPMC 118 may signal the completion of the operation to programmer 112. Preferably, the DRPMC 118 will then close the connection with programmer 112, for example after a disconnect request to the programmer 112, for example, as part of the implementation of a symmetric release to avoid possible loss of data. The programmer 112 may then terminate the telemetry or other wireless connection with the IMD 111. This may involve the issuance of instructions to a human programmer Operator, if applicable, to effect the termination in a certain manner, for example, by removing the programmer 112 from proximity to the host patient. Preferably, the programmer 112 will not terminate communication with the IMD 111 until after the connection with the DRPMC 118 is released.

In a preferred embodiment of the subject invention, communication system 110 will operate asynchronously, permitting for the possibility for breaks in the continuous and real-time communications and/or processing of the three subsystems (IMD 111, programmer 112, and DRPMC 118). However, alternative embodiments of the invention are also possible, including synchronous, "real-time" control of the target IMD 111. This alternative "real-time" embodiment of the system 110 may be enhanced upon the establishment of more ubiquitous and robust communications systems or links.

Initially the system would act in an asynchronous manner, where precise timing of data transfer and therapy changes is not critical. As the device-instrument and network communications become more ubiquitous and less reliant on specific hardware (e.g. RF head, network cables), the control loop could become more time-dependent.

Although the invention is described with reference to particular embodiments, it will be understood to those skilled in the art that this embodiment is merely illustrative of the application of the principles of the invention.

## Claims

1. A computerized information network system (110) comprising one or more IMD programmers (112), a centralized computer system, a data communication network linking said IMD programmer(s) to said centralized computer system, said network system further comprising:
a first computer (118) accessible by the data communication network, adapted to store data regarding an IMD including information about implantation of the IMD,
at least one network interface to at least one programmer, said network interface being capable of communication with the data communication network;
**characterized by** at least one additional peripheral computer (120) in data communication with the first computer, the at least one additional peripheral computer being programmed to process internal accounting and finance data based on said information about implantation of the IMD.

2. The computerized network system of claim 1, wherein the data communication network comprises a link between the at least one network interface and the centralized computer system.

3. The computerized network system of claim 1 or 2, wherein the first computer comprises a multi-processor workstation.

4. The computerized network system of claim 1 or 2, wherein the first computer comprises a networked or parallel cluster of computers.

5. The computerized network system of any preceding claim, wherein the data communication is asynchronous.

6. The computerized network system of any of claims 1 to 4, wherein the data communication is synchronous.

7. The computerized network system of any preceding claim, wherein the centralized computer system is adapted to generate and transmit electronically an invoice pertaining to an IMD programmer event.

8. The computerized network system of any preceding claim, further comprising at least one additional peripheral computer adapted to generate an invoice.

## Patentansprüche

1. Computergesteuertes Informationsnetzwerksystem (110) mit einem oder mehreren IMD-Programmgebern (112), einem zentralen Computersystem, einem Datenkommunikationsnetzwerk, das den/die IMD-Programmgeber mit dem zentralen Computersystem verbindet, wobei das Netzwerksystem des weiteren folgendes umfasst:
einen ersten Computer (118), auf den von dem Datenkommunikationsnetzwerk zugegriffen werden kann und der dazu ausgelegt ist, Daten betreffend ein IMD einschließlich Informationen zur Implantation des IMD zu speichern,
mindestens eine Netzwerkschnittstelle zu einem Programmgeber, wobei die Netzwerkschnittstelle dazu in der Lage ist, mit dem Datenkommunikationsnetzwerk zu kommunizieren,
**gekennzeichnet durch** mindestens einen peripheren Computer (120), der in Datenkommunikation mit dem ersten Computer steht, wobei der mindestens eine zusätzliche periphere Computer dazu programmiert ist, interne Buchhaltungs- und Finanzdaten basierend auf den Informationen über die Implantation des IMD zu verarbeiten.

2. Computergesteuertes Netzwerksystem nach Anspruch 1, wobei das Datenkommunikationsnetzwerk eine Verbindung zwischen der mindestens einen Netzwerkschnittstelle und dem zentralen Computersystem umfasst.

3. Computergestütztes Netzwerksystem nach Anspruch 1 oder 2, wobei der erste Computer einen Mehrfachprozessor-Arbeitsplatz umfasst.

4. Computergestütztes Netzwerksystem nach Anspruch 1 oder 2, wobei der erste Computer einen vernetzten oder parallelen Cluster von Computern umfasst.

5. Computergestütztes Netzwerksystem nach einem der voranstehenden Ansprüche, wobei die Datenkommunikation asynchron ist.

6. Computergestütztes Netzwerksystem nach einem der Ansprüche 1 bis 4, wobei dei Datenkommunikation synchron ist.

7. Computergestütztes Netzwerksystem nach einem der voranstehenden Ansprüche, wobei das zentrale Computersystem dazu angepasst ist, elektronisch eine einen IMD-Programmgeber-Anlass betreffende Rechnung zu erstellen und zu übertragen.

8. Computergestütztes Netzwerksystem nach einem der voranstehenden Ansprüche, das des weiteren mindestens einen zusätzlichen peripheren Computer umfasst, der dazu ausgelegt ist, eine Rechnung zu erzeugen.

## Revendications

1. Système de réseau d'informations informatisé (110) comportant un ou plusieurs programmateurs de dispositifs médicaux implantables (IMD) (112), un système informatique centralisé, un réseau de communication de données reliant le ou lesdits programmateurs de IMD audit système informatique centralisé, ledit système de réseau comportant de plus :
un premier ordinateur (118) accessible par le réseau de communication de données, adapté pour mémoriser des données concernant un dispositif IMD incluant des informations se rapportant à l'implantation du dispositif IMD,
au moins une interface réseau avec au moins un programmateur, ladite interface réseau étant capable de communiquer avec le réseau de communication de données,
**caractérisé par** au moins un ordinateur périphérique supplémentaire (120) en communication de données avec le premier ordinateur, le au moins un ordinateur périphérique supplémentaire étant programmé pour traiter des données de comptabilité et de finances internes sur la base desdites informations concernant l'implantation du dispositif IMD.

2. Système de réseau informatisé selon la revendication 1, dans lequel le réseau de communication de données comporte une liaison entre la au moins une interface réseau et le système informatique centralisé.

3. Système de réseau informatisé selon la revendication 1 ou 2, dans lequel le premier ordinateur constitue un poste de travail multiprocesseur.

4. Système de réseau informatisé selon la revendication 1 ou 2, dans lequel le premier ordinateur constitue une grappe d'ordinateurs en réseau ou parallèles.

5. Système de réseau informatisé selon l'une quelconque des revendications précédentes, dans lequel la communication de données est asynchrone.

6. Système de réseau informatisé selon l'une quelconque des revendications 1 à 4, dans lequel la communication de données est synchrone.

7. Système de réseau informatisé selon l'une quelconque des revendications précédentes, dans lequel le système informatique centralisé est adapté pour générer et transmettre électroniquement une facture se rapportant à un événement de programmateur de IMD.

8. Système de réseau informatisé selon l'une quelconque des revendications précédentes, comportant de plus au moins un ordinateur périphérique supplémentaire adapté pour générer une facture.
